# EUROPEAN PATENT SPECIFICATION

(11) **EP 1 537 825 B1**
(45) Date of publication and mention of the grant of the patent: **05.03.2008**
(21) Application number: 04257484.8
(22) Date of filing: 01.12.2004
(51) Int. Cl.: A61B 6/03

(54) **Cooling and power system for a medical imaging system**
Kühl- und Leistungssystem für eine medizinische Abbildungsanordnung
Système de puissance et de refroidissement pour un appareil d'imagerie médicale

(30) Priority: 01.12.2003 US 725302
(43) Date of publication of application: 08.06.2005
(73) Proprietor: GE Medical Systems Global Technology Company LLC, Waukesha, Wisconsin 53188-1696 (US)
(72) Inventor: Powell, David L., North Salt Lake, Utah 84504 (US)
(74) Representative: Goode, Ian Roy

(56) References cited:
- EP-A- 0 404 335
- EP-A- 0 920 241
- US-A- 4 115 697
- US-A- 5 438 605
- US-A- 5 610 968
- US-B1- 6 412 979

## Description

Embodiments of the present invention generally relate to a cooling and/or power system for a medical imaging system, and more particularly to an auxiliary module for cooling, and/or providing power to, an x-ray imaging system.

X-ray imaging systems typically include an x-ray tube, a detector, and a positioning arm, such as a C-arm, supporting the x-ray tube and the detector. In operation, an imaging table, on which a patient is positioned, is located between the x-ray tube and the detector. The x-ray tube typically emits radiation, such as X-rays, toward the patient. The radiation typically passes through the patient positioned on the imaging table and impinges on the detector. As the radiation passes through the patient, anatomical structures inside the patient cause spatial variances in the radiation received at the detector. The detector then translates the radiation variances into an image, which may be employed for clinical evaluations.

X-rays are produced when high-speed electrons are suddenly decelerated, for example, when a metal target, is struck by electrons that have been accelerated through a potential difference of several thousand volts. Typically, x-ray emitters include an anode, which may be fixed or rotatable, and a cathode. If the anode is rotatable, the anode may be rotated at a high rate of speed in order to manage the resulting heat on the target from the cathode during the x-ray emission process.

Some procedures require extended periods of imaging, such as through x-rays, in order to properly diagnose, treat, and/or assess the condition of, a patient. Often, during extended imaging, the imaging element, such as an x-ray tube, overheats such that an operator has to interrupt the imaging procedure in order to allow the imaging element to cool. In mobile x-ray systems in particular, fans and rotating elements within the x-ray tube may not adequately dissipate the heat produced by the x-ray tube. Thus, imaging may be interrupted for extended periods of time. Further, during periods of extended imaging, the imaging system may not be supplied with adequate power. That is, during periods of extended imaging, power levels within the imaging device may be depleted to the point in which further imaging is precluded.

Thus, a need exists for a more efficient system and method of cooling an imaging element of a medical imaging system. Additionally, a need exists for a system and method for providing additional power to a medical imaging system during periods of intense and/or extended imaging. Document US-A-6 412 979 discloses a computed tomography system with arrangement for cooling the X-ray radiator.

According to the present invention there is provided a medical imaging system a defined in claim 1, preferably including a medical imaging device having a main body and an imaging element, and an auxiliary module having a cooling unit configured to circulate chilled liquid to and from the imaging element. As the chilled liquid circulates or passes around the imaging element, the chilled liquid absorbs heat produced by the imaging element during an imaging procedure. The imaging element may be an x-ray tube, included within a mobile x-ray C-arm device.

A cooling duct surrounds at least a portion of the imaging element. The cooling duct includes a fluid inlet and a fluid outlet, which are in fluid communication with a fluid input line (or supply tube) and a fluid return line (or return tube), respectively. The chilled liquid is supplied to the cooling duct from the cooling unit through the fluid input line. The chilled liquid is returned to the cooling unit through the fluid return line. The cooling unit includes a pump, or other such component, which acts to circulate the chilled liquid between the cooling unit and the cooling duct.

The cooling duct may be permanently or removably connected to the imaging element. For example, the cooling duct may be configured to be positioned over the imaging element during periods of imaging; but, when the imaging device is not in operation, the cooling duct may be removed from the imaging element and stored with the auxiliary module. The auxiliary module may be mobile and/or remotely located from the imaging device. For example the auxiliary module may include a cart having wheels (e.g., caster assemblies) that allow it to be conveniently moved between various locations. Optionally, the auxiliary module may be permanently fixed to the imaging device, or another structure such as a wall, a floor, or another stationary structure.

The auxiliary module also includes a booster battery pack, which is configured to be electrically connected to the medical imaging device in order to provide additional power to the medical imaging device during periods of intense and/or extended imaging. The main body of the imaging device includes a power boost receptacle electrically connected to a power supply system within the imaging device. A power cable electrically connected to the booster battery pack may be removably connected (e.g., a plug and socket relationship) to the power boost receptacle so that the power supply system may draw power from the booster battery pack.

Embodiments of the invention will now be described, by way of example, with reference to the accompanying drawings, in which:
Figure 1 illustrates a medical imaging system according to an embodiment of the present of the present invention.
Figure 2 illustrates an interior side view of an x-ray tube according to an embodiment of the present invention.

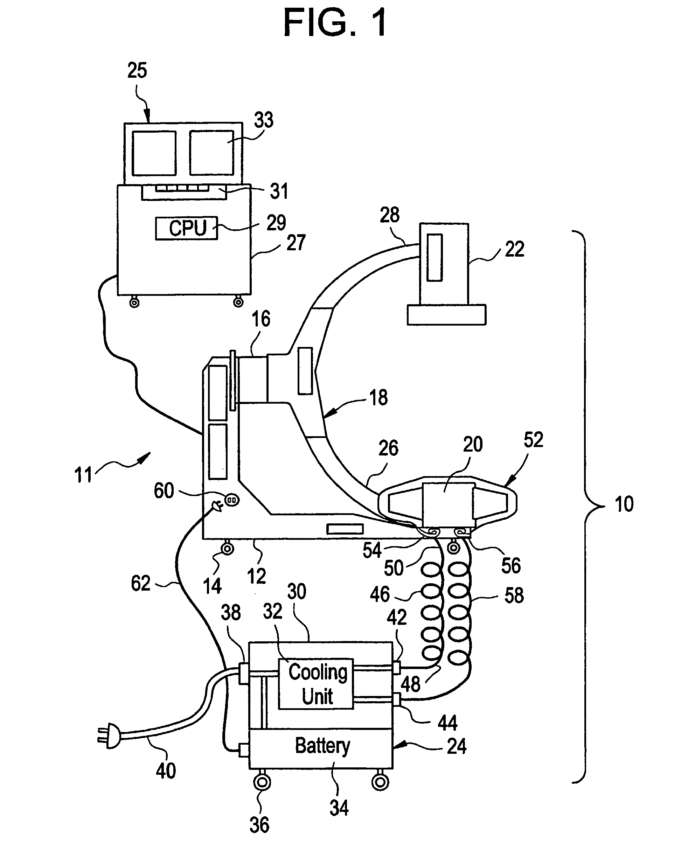

Figure 1 illustrates a medical imaging system 10 according to an embodiment of the present invention. The medical imaging system 10 includes an x-ray device 11, which includes a base member 12 having wheels 14 (e.g., caster assemblies), a main body 16, a positioning arm 18, such as a C arm, an x-ray tube 20, and a detector 22. The medical imaging system 10 also includes an auxiliary module, such as a remote cooling and power system, 24 and a workstation 25 operatively connected to the x-ray device 11. The positioning arm 18 includes a first end, or first prong 26, and a second end, or second prong 28.

The base member 12 supports the entire structure of the x-ray device 11. The base member 12 is integrally connected to the main body 16, which is in turn connected to the positioning arm, or C-arm 18. The x-ray tube 20 is located at a distal end of the first prong 26, while the detector 22 is located at a distal end of the second prong 28. The x-ray tube 20 and the detector 22 are oriented such that the x-ray tube 20 emits radiation toward the detector 22. Examples of x-ray C-arms are further described in United States Patent No. 6,104,780, entitled "Mobile bi-planar fluoroscopic imaging apparatus," United States Patent No. 5,802,719, entitled "One piece C-arm for x-ray diagnostic equipment," and United States Patent No. 5,627,873, entitled "Mini C-arm assembly for mobile x-ray imaging system".

The x-ray device 11 is also operatively connected to a workstation 25. The workstation 25 may be a mobile workstation, such that it may be moved between various locations. The workstation 25 includes a housing 27 that supports a central processing unit (CPU) 29, an input device 31 (such as a keyboard, mouse, or touch sensitive monitor), and a display unit 33. The input device 31 and the display unit 33 are in electrical communication with the CPU 29. The workstation 25 allows a user to operate the x-ray device 11 and monitor the operation thereof.

In operation, a patient is positioned on an x-ray positioning table (not shown) between the x-ray tube 20 and the x-ray detector 22. After the patient is positioned, the imaging process may begin. To begin the imaging process, the x-ray tube 20 is activated. During imaging, the x-ray tube 20 emits radiation that passes through the patient and is received by the detector 22.

The auxiliary module 24 includes a cart 30 housing a cooling unit 32 and a booster battery pack 34. The cooling unit 32 may be a cold plate, an ice reservoir, a fluid condensing/evaporating system such as in an air conditioning and/or refrigeration unit, a series of heat transfer tubes and pipes, thermionic cooling devices, or various other known cooling systems that are capable of cooling and circulating fluid. The cart 30 is supported by wheels 36, e.g., caster wheel assemblies, such that the auxiliary module 24 may be conveniently moved to and from various imaging or operating environments. A power input 38 operatively connected to a power cable 40 provides electrical power to the cooling unit 32 (in order to operate the cooling unit 32) and the booster battery pack 34 (in order to recharge the booster battery pack 34 with electrical power). The power cable 40 may be removably connected to a source of power, such as a standard AC current wall input. Optionally, the power cable 40 may be removably connectable to various other known sources of power known in the art.

The cooling unit 32 is operatively connected to a cooling fluid output 42 and a return fluid input 44. The cooling unit 32 is configured to pump, or otherwise supply, cooled (i.e., chilled) fluid from the cooling unit 32 through the cooling fluid output 42. Further, the cooling unit 32 is configured to receive fluid through the return fluid input 44. The fluid may be water, oil, antifreeze, or various other appropriate liquids that may be circulated within the x-ray tube 20.

A tube 46 is operatively connected to the fluid output 42 at a first end 48 such that chilled fluid may pass from the cooling unit 32, through the cooling fluid output 42, and into the tube 46. A second end 50 of the tube 46 is removably connected to a cooling duct 52, by way of a fluid inlet 54, positioned around the x-ray tube 20. The cooling duct 52 may be an air/fluid-tight tube-like structure that overlies a portion of the x-ray tube 20. Optionally, the cooling duct 52 may be an air/fluid-tight membrane, sack or pouch that surrounds all, or substantially all, of the x-ray tube 20. The cooling duct 52 also includes a fluid outlet 56. A tube 58 is removably connected to the fluid outlet 56 to allow fluid to pass from the cooling duct 52 back to the cooling unit 32 through the return fluid input 44. The fluid outlets 48, 56 and the fluid inlets 44 and 54 include structures, such as check valves, to ensure that fluid does not escape when the cooling unit 32 is not in operation and/or when the tubes 46 and 58 are not connected thereto. Prior to imaging, the tubes 46 and 56 are connected to the fluid inlets 54 and fluid outlet 56, respectively, so that the x-ray tube 20 may be cooled by chilled fluid circulated by the cooling unit 32.

As shown in Figure 1, the tubes 46 and 58 are removably connected to the cooling duct 52 at the fluid inlet 54 and outlet 56, respectively. Alternatively, the fluid inlet 54 and the fluid outlet 56 may be positioned on the main body 16 of the x-ray imaging device 11. Pipes, ducts, or other such structures may be positioned within the main body 12 and the positioning arm 18 in order to allow fluid to pass from the cooling unit 32 to the cooling duct 52. That is, the x-ray imaging device 11 may include interior piping, ducts, passages, channels and the like that are adapted to allow fluid to pass between the cooling duct 52 and the cooling unit 32. Also, alternatively, the x-ray imaging device 11 may include exterior piping, ducts, passages, channels and the like that are adapted to allow fluid to pass between the cooling duct 52 and the cooling unit 32. Thus, instead of constructing the x-ray imaging device 11 to have channels, ducts, and the like within the x-ray imaging device 11, these components may be positioned on the outside of the main body 16 and the positioning arm 18.

Also, alternatively, the cooling duct 52 may not be permanently attached to the x-ray tube 20. Instead, the tubes 46, 56 and the cooling duct 52 may be a single, unitary, integrally-formed structure. In this case, the fluid inlet 54 and fluid outlet 56 are unnecessary due to the fact that the tubes 46, 58 and cooling duct 52 are a unitary structure. The structure defined by the tubes 46, 58 and the cooling duct 52 may be configured to be removably wrapped, draped, or otherwise positioned over, the x-ray tube 20. Thus, when the x-ray imaging device 11 is not in operation, the cooling structure is removed from the x-ray tube 20. Prior to imaging, however, the cooling structure may be operatively connected to (e.g., draped, shrouded, enveloped, etc., around) the x-ray tube 20. Optionally, a removable cooling structure may be positioned within the x-ray tube 20, while another removable cooling structure may be positioned outside the x-ray tube 20. Further, the removable cooling structure may include a portion positioned within the x-ray tube 20 and another portion positioned outside the x-ray tube 20.

The x-ray imaging device 11 also includes a power boost receptacle 60. Further, the booster battery pack 34 is electrically connected to a power cable 62 that extends from the cart 30. The booster battery pack 34 may include various types of batteries, such as are known in the art, depending on the amount of power required for a certain imaging session. The booster battery pack 34 may include one or more primary and/or secondary power sources. For example, the booster battery pack 34 may include one or more of the following battery types: Nickel Metal Hydride; Nickel Cadmium; Lead-Acid; Lithium Ion; Lithium Polymer; Sodium Nickel Chloride; Zinc-Air; Vanadium Redox; Carbon-Zinc; Zinc-Chloride; Alkaline; Mercuric Oxide; Zinc Bromide; and/or various other battery types known in the art.

The booster battery pack 34 may be electrically connected to the main power system (not shown) of the x-ray imaging device 11 through the interface between the power cable 62 and the power boost input 60. The booster battery pack 34 is configured to supply additional power to the x-ray imaging device 11 for extended periods of imaging and/or particularly intense imaging. The booster battery pack 34 is adapted to be charged and recharged through the interface between the power cable 40 and a source of power, such as a through standard AC current wall input. While the x-ray imaging device 11 is typically connected to a source of power separate and distinct from the booster battery pack 34, the x-ray imaging device 11 may also be powered solely through the booster battery pack 34 in order to facilitate imaging in an environment where power receptacles are scarce (e.g., operatively connected to other devices). Further, the booster battery pack 34 may also be used to supply power to operate the cooling unit 32.

Additionally, the workstation 25 may be electrically connected to the auxiliary module 24 to monitor characteristics of the auxiliary module 24. For example, the CPU 29 may be configured to receive data from the auxiliary unit 24 regarding cooling liquid temperature, battery power level, and the like. The CPU 29 may then display information regarding these characteristics to an operator on the display 33. Further, the CPU 29 may automatically operate the auxiliary unit 24 based on predetermined parameters. For example, if the cooling fluid within the auxiliary module 24 is low, or is too warm, the CPU 29 may cease operation of the auxiliary module 24 and/or the x-ray device 11. Once fluid levels reach an acceptable level (in terms of amount and temperature), the CPU 29 may activate use of the auxiliary module 24 and/or x-ray device 11.

Additionally, the x-ray device 11 may include sensors (not shown) that measure power levels and the like. The CPU 29 may monitor sensed power levels to determine whether additional power from the auxiliary module 24 is needed. The CPU 29 may automatically direct the auxiliary module 24 to provide additional power to the x-ray device 11, or the CPU 29 may display a message on the display 33 alerting an operator that additional power is needed. Similarly, the temperature of the x-ray tube 20 may be monitored by a thermometer (not shown), or other such device, which is in electrical communication with the CPU 29 or a processing unit within the x-ray device 11 itself. Data regarding x-ray tube temperature may be relayed to the workstation 25, where it is subsequently displayed.

While the imaging system 11 is shown as a mobile imaging system, embodiments of the present invention may also be used with permanent, non-mobile imaging systems. For example, the auxiliary module 24 may be used with a fixed x-ray C-arm imaging system. Optionally, the auxiliary module 23 may not be mobile, but may rather be mounted to a wall, floor, or a structure within an imaging room. Further, embodiments of the present invention may provide a cooling and/or power boost system that is not mobile. The cooling and/or power boost system may be affixed to the main body 16 or the positioning arm 18, instead of being remotely located thereform.

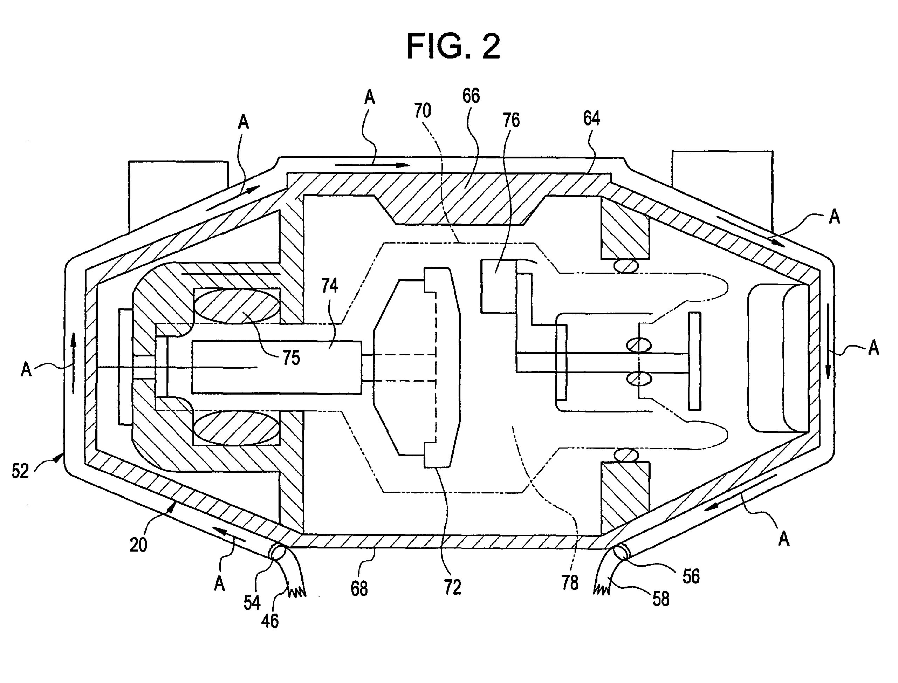

Figure 2 illustrates an interior side view of the x-ray tube 20. The x-ray tube 20 includes a casing 64, a radiation emission passage 66 formed within the casing 64 and a mounting plate 68 on the side of the x-ray tube 20 opposite that of the emission passage 66. The casing 64 encloses an insert, or emitter 70, which houses the internal components of the x-ray tube 20. The internal components include an anode 72, an extension rod 74, such as an axle, a cathode 76, and a bearing 75 within a vacuum 78. The cathode 76 and anode 72 are mounted within the interior of the insert 70. The anode 72 connects to the extension rod 74. The opposite end of the extension rod 74 is retained by the bearing 75. The bearing is in turn retained by an additional structure, such as a motor mounted within the insert 70. The insert 70 is in turn mounted within the casing 64. While the x-ray tube 20 includes a rotatable anode 72, the x-ray tube 20 may alternatively include a fixed anode.

As mentioned above, x-rays are produced when high-speed electrons are suddenly decelerated, for example, when a metal target, i.e., the anode 72, is struck by electrons that have been accelerated through a potential difference of more than 80 thousand volts. The x-rays are then emitted through the radiation emission passage 66 toward the detector 22. As stated above, the anode 72 is connected to the axle, i.e., extension rod 74. The extension rod 74 is rotated through the bearing 75, which is activated by the motor. The rotation of the bearing 75 causes the extension rod 74 to rotate. The rotation of the extension rod 74 causes the anode 72 to rotate. The bearing 75 rotates the anode 72 at a high rate, for example 125 Hz.

The cooling unit 32 circulates chilled fluid through the cooling duct 52 in the direction of arrow A. As discussed above, chilled fluid is passed into the cooling duct 52 through the tube 46 and fluid inlet 54. As the fluid passes through the cooling duct 52, the fluid absorbs heat produced within the x-ray tube 20. Heat flows from hotter, i.e., higher energy, sources, to cooler, i.e., lower energy sources. Thus, heat produced within the x-ray tube 20 radiates and/or conducts outwardly toward the chilled water within the cooling duct 52. As the chilled fluid absorbs heat, the temperature of the chilled fluid increases. The fluid is passed through the cooling duct 52 through the fluid outlet 56 and into the tube 58. The fluid is then returned to the cooling unit 32, which re-chills the fluid so that it may be re-circulated into the cooling duct 52. Thus, embodiments of the present invention provide a system and method for cooling the x-ray tube 20.

While the cooling duct 52 is shown outside of the casing 64, the cooling duct 52 may optionally be located within the interior of the x-ray tube 20 under the casing 64. Additionally, instead of being in direct contact with the casing 64, the cooling duct 52 may be positioned directly over the emitter 70, or other components of the x-ray tube 20.

The cooling and power boost systems discussed above may be used with other imaging devices. For example, embodiments of the present invention may be used with ultrasound systems to cool ultrasound probes and provide additional power thereto. Overall, embodiments of the present invention may be used with any system in which heat is produced as a by-product of the imaging process and/or a system that may benefit from additional power.

For example, embodiments of the present invention may be used with various imaging modalities, such as Computed Tomography (CT), X-ray (film-based and digital x-ray systems), Positron Emission Tomography (PET), such as shown and described in United States Patent No. 6,337,481, entitled "Data binning method and apparatus for PET tomography including remote services over a network." Single Photon Emission Computed Tomography (SPECT), such as shown and described in United States Patent No. 6,194,725, entitled "SPECT system with reduced radius detectors", Electron Beam Tomography (EBT), such as shown and described in United States Patent No. 5,442,673, entitled "Fixed septum collimator for electron beam tomography," and Magnetic Resonance (MR), such as described in United States Patent No. 6,462,544, entitled "Magnetic resonance imaging apparatus," and various other imaging systems. Additionally, embodiments of the present invention may also be used with navigation and tracking systems, such as those described in United States Patent No. 5,803,089, entitled "Position Tracking and Imaging System for Use in Medical Applications".

Thus, embodiments of the present invention provide an efficient system of cooling imaging elements. Further, embodiments of the present invention provide a system of supplying additional power to an imaging system.

## Claims

1. A medical imaging system (10), comprising:
a medical imaging device (11) having a main body and an imaging element (20); and
an auxiliary module (24) configured to cool the imaging element and supply additional power to the medical imaging device, the auxiliary module (24) comprising:
a cooling unit (32) configured to circulate chilled liquid to and from the imaging element (20), wherein the chilled liquid absorbs heat produced by the imaging element; and
a booster battery pack (34), wherein said booster battery pack (34) is configured to be electrically connected to the medical imaging system (10) in order to provide additional power to the medical imaging system (10);
the cooling unit (32) and the battery pack (34) being removably connected to the medical imaging device (11).

2. The medical system of claim 1 including a central processing unit (29) for monitoring characteristics of the auxiliary module (24) and automatically operating the auxiliary module based on predetermined parameters including chilled liquid temperature and battery power level.

3. The medical imaging system (10) of claim 1, wherein said imaging element includes an x-ray tube (20), wherein said cooling unit (32) circulates the chilled liquid over, or within, said imaging element.

4. The medical imaging system (10) of claim 3, further comprising a C-arm (18) supported by said main body, wherein said x-ray tube (20) is positioned on an end of said C-arm (18).

5. The medical imaging system (10) of claim 1, further comprising:
a cooling duct (52) surrounding at least a portion of said imaging element, said cooling duct (52) having a fluid inlet (54) and an fluid outlet (56);
a fluid input line in fluid communication with said cooling unit (32) and said fluid inlet (54), wherein the chilled liquid is supplied to said cooling duct (52) from said cooling unit (32) through said fluid input line; and
a fluid return line in fluid communication with said cooling unit (32) and said fluid outlet (56), wherein the chilled liquid is returned to said cooling unit (32) through said fluid return line.

6. The medical imaging system (10) of claim 1, wherein said auxiliary module (24) is mobile.

## Patentansprüche

1. Medizinische Bildgebungs-Vorrichtung (10), die aufweist:
eine medizinische Bildgebungs-Einrichtung (11), die einen Hauptkörper und ein Bildgebungs-Element (20) aufweist; und
ein Hilfsmodul (24), das eingerichtet ist, das Bildgebungs-Element zu kühlen und zusätzliche Leistung für die medizinische Bildgebungs-Einrichtung bereitzustellen, wobei das Hilfsmodul (24) aufweist:
einen Kühleinheit (32), die dazu eingerichtet ist, eine gekühlte Flüssigkeit zu und von dem Bildgebungs-Element (20) weg zirkulieren zu lassen, wobei die gekühlte Flüssigkeit Wärme absorbiert, die durch das Bildgebungs-Element erzeugt wird; und
eine Batterie (34), wobei die Batterie (34) eingerichtet ist, um elektrisch mit der medizinischen Bildgebungs-Vorrichtung (10) verbunden zu sein, um zusätzliche Leistung für die medizinische Bildgebungs-Vorrichtung (10) bereitzustellen;
wobei die Kühleinrichtung (32) und die Batterie (34) lösbar mit der medizinischen Bildgebungs-Einrichtung (11) verbunden sind.

2. Medizinische Vorrichtung nach Anspruch 1, die ferner eine zentrale Bearbeitungseinheit (29) zum Überwachen der Eigenschaften des Hilfsfmoduls (24) enthält, und die automatisch das Hilfsmodul auf der Basis von vorbestimmten Parametern betreibt, zu denen die Temperatur der gekühlten Flüssigkeit und der Spannungs-Pegels der Batterie gehören.

3. Medizinische Bildgebungs-Vorrichtung (10) nach Anspruch 1, worin das Bildgebungs-Element eine Röntgenröhre (20) enthält, worin die Kühleinheit (32) die gekühlte Flüssigkeit über oder innerhalb des Bildgebungs-Elementes zirkulieren lässt.

4. Medizinische Bildgebungs-Vorrichtung (10) nach Anspruch 3, die ferner einen C-Arm (18) aufweist, der durch den Hauptkörper gehalten wird, worin die Röntgenröhre (20) auf einem Ende des C-Arms (18) positioniert ist.

5. Medizinische Bildgebungs-Vorrichtung (10) nach Anspruch 1, die ferner aufweist:
eine Kühl-Rohrleitung (52), die mindestens einen Bereich des Bildgebungs-Elements umgibt, wobei die Kühl-Rohrleitung (52) einen Flussigkeits-Einlass (54) und einen Flüssigkeits-Auslass (56) aufweist;
eine Flüssigkeits-Einlass-Leitung in Strömungsverbindung mit der Kühleinheit (32) und dem Flüssigkeits-Einlass (54) steht, worin die gekühlte Flüssigkeit zu der Kühl-Rohrleitung (52) von der Kühleinheit (32) durch die Flüssigkeits-Einlass-Leitung bereitgestellt wird, und
eine Flüssigkeits-Rückflussleitung in Strömungsverbindung mit der Kühleinheit (32) und dem Flüssigkeits-Auslass (56) steht, worin die gekühlte Flüssigkeit zu der Kühleinheit (32) durch die Flüssigkeits-Rückflussleitung zurückfließt.

6. Medizinische Bildgebungs-Vorrichtung (10) nach Anspruch 1, worin das Hilfsmodul (24) beweglich ist.

## Revendications

1. Système (10) d'imagerie médicale comprenant :
un dispositif (11) d'imagerie médicale ayant un corps principal et un élément (20) d'imagerie ; et
un module auxiliaire (24) configuré pour refroidir l'élément d'imagerie et fournir de la puissance supplémentaire au dispositif d'imagerie médicale, le module auxiliaire (24) comprenant :
une unité (32) de refroidissement configuré pour faire circuler le liquide refroidi vers et à partir de l'élément (20) d'imagerie, dans laquelle le liquide refroidi absorbe la chaleur produite par l'élément d'imagerie ; et
un bloc (34) de batterie de démarrage, dans lequel ledit bloc (34) de batterie de démarrage est configuré pour être connecté électriquement au système (10) d'imagerie médicale afin de fournir de la puissance supplémentaire au système (10) d'imagerie médicale ;
l'unité (32) de refroidissement et l'ensemble (34) de batterie étant connectés de manière amovible au dispositif (11) d'imagerie médicale.

2. Système médical selon la revendication 1 comprenant une unité (29) de traitement central pour surveiller les caractéristiques du module auxiliaire (24) et faire fonctionner automatiquement le module auxiliaire en se basant sur des paramètres prédéterminés comprenant la température du liquide refroidi et le niveau de puissance de la batterie.

3. Système (10) d'imagerie médicale selon la revendication 1, dans lequel ledit élément d'imagerie comprend un tube (20) à rayons X, dans lequel ladite unité (32) de refroidissement fait circuler le liquide refroidi sur, ou à l'intérieur dudit lément d'imagerie.

4. Système (10) d'imagerie médicale selon la revendication 3, comprenant en outre un bras en C (18) soutenu par ledit corps principal, dans lequel ledit tube (20) à rayons X est positionné sur une extrémité dudit bras en C (18).

5. Système (10) d'imagerie médicale selon la revendication 1, comprenant en outre :
un conduit (52) de refroidissement entourant au moins une partie dudit élément d'imagerie, ledit conduit (52) de refroidissement ayant une entrée (54) de fluide et une sortie (56) de fluide ;
une ligne d'entrée de fluide dans la communication de fluide avec ladite unité (32) de refroidissement et ladite entrée (54) de fluide, dans laquelle le liquide refroidi est fourni audit conduit (52) de refroidissement à partir de l'unité (32) de refroidissement à travers ladite ligne d'entrée de fluide ; et
une conduite de retour de fluide dans la communication de fluide avec ladite unité (32) de refroidissement et ladite sortie (56) de fluide, dans laquelle le liquide refroidi est retourné à ladite unité (32) de refroidissement à travers ladite conduite de retour de fluide.

6. Système (10) d'imagerie médicale selon la revendication 1, dans lequel ledit module auxiliaire (24) est mobile.
